(19) Europäisches Patentamt
European Patent Office
Office européen des brevets

(11) Publication number: **0 071 357**
**B1**

(12) # EUROPEAN PATENT SPECIFICATION

(45) Date of publication of patent specification: **02.01.85**

(21) Application number: **82303641.3**

(22) Date of filing: **12.07.82**

(51) Int. Cl.⁴: **A 61 K 31/20,** A 61 K 31/23, A 61 K 45/02 // (A61K31/23, 31/20, 31/07),(A61K45/02, 33/30, 31/485, 31/475, 31/425, 31/40, 31/43, 31/375, 31/23, 31/20, 31/165, 31/13, 31/07, 31/045)

(54) **Pharmaceutical and dietary composition when used for enhancement of 1-series PG production.**

(30) Priority: **14.07.81 GB 8121668**

(43) Date of publication of application:
**09.02.83 Bulletin 83/06**

(45) Publication of the grant of the patent:
**02.01.85 Bulletin 85/01**

(84) Designated Contracting States:
**AT BE CH DE FR GB IT LI LU NL SE**

(56) References cited:
**EP-A-0 019 423**

**CHEMICAL ABSTRACTS, vol. 70, no. 18, 5th May 1969, page 252, column 2, no. 80843k, Columbus Ohio (USA);**

(73) Proprietor: **EFAMOL LIMITED**
**71/74 Mark Lane**
**London E.C.3 (GB)**

(72) Inventor: **Horrobin, David Frederick**
**P.O. Box 10**
**Nuns Island Montreal H3E 1J8 (CA)**

(74) Representative: **Caro, William Egerton et al**
**J. MILLER & CO. Lincoln House 296-302 High Holborn**
**London WC1V 7JH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 071 357 B1

## Description

Field of the invention

This invention relates to the treatment of certain diseases and disorders primarily, but not exclusively, in the field of human medicine and to compositions for use therein.

General background

Considerable interest has been shown in recent years in the use of prostaglandin (PG) precursors in medicine.

For various reasons it is not practical to administer naturally-occurring prostaglandins such as PGE 1 and PGE 2 to patients. Consequently, considerable attention has focussed on the use of prostaglandin precursors including linoleic acid, γ-linolenic acid (GLA) and dihomo-γ-linolenic acid (DGLA).

Conversion of these materials in the body is believed to be as shown in the following diagram (see annexed sheet).

The broad outline of this pathway is well known, and it brings out clearly that a major function of essential fatty acids (EFAs) is to act as precursors for prostaglandins, 1-series PGs being formed from dihomo-γ-linolenic acid (DGLA) and 2-series PGs from arachidonic acid (AA). DGLA and AA are present in food in only small quantities, and the major EFA in food is linoleic acid which is first converted to γ-linolenic acid (GLA) and then to DGLA and AA. The conversion of linoleic acid to GLA is blocked by a high fat and high carbohydrate diet, by ageing and for example by diabetes. Stores of AA in the body in the form of lipid esters are very large indeed. In contrast only small amounts of DGLA ester are present.

Invention and detailed background

DGLA is the key substance. GLA is almost completely and very rapidly converted in the body to DGLA and so for practical purposes the oral administration of DGLA and GLA amounts to the same thing. DGLA can be converted to a storage form, changed to arachidonic acid and thence to PGs of the 2 series, or converted to PGs of the 1 series.

There is increasing evidence that PGs of the 1 series play a vital role in a number of key areas. First, PGE 1 activates T lymphocytes. Defective T lymphocytes are believed to be involved in causing a wide range of allergic and inflammatory disorders and in making individuals susceptible to cancer and infections of all types. Second, PGE 1 is important in preventing over-production of collagen and fibrous tissue, a factor which plays a major role in arthritis and the so-called collagen diseases. Third, PGE 1 levels are extremely low in patients with schizophrenia and are moderaly low in patients with depression. Fourth, PGE 1 appears to be important in controlling cholesterol levels and necessary for the normal actions of insulin. Fifth, PGE 1 dilates blood vessels and may be expected to be helpful in any situation in which vessel spasm occurs. Sixth, PGE 1 appears to inhibit the production of 2-series PG's, levels of which are raised in a wide variety of inflammatory disorders. Seventh, PGE 1 increases production of cyclic AMP which has anti-inflammatory effects.

There are therefore very strong reasons, and thus broadly is an aim of the present invention, for influencing the 1-series/2-series PG balance in the body in favour of 1-series PG's and specifically for selectively enhancing formation of PGs of the 1-series and particularly PGE 1. The diseases and disorders below are among those in which such action is indicated:—

1. Defective T lymphocyte function inlcuding such defective function as allergic and inflammatory disorders, multiple sclerosis, schizophrenia and cancer.

2. Defective regulation of collagen formation and breakdown including such defective regulation as including rheumatoid arthritis, systemic lupus erythematosus, Crohn's disease and the "collagen" diseases.

3. Mental illnesses showing low PGE 1 levels including depression, schizophrenia and alcohol withdrawal and the latter stages of alcohol intoxication. In depression for example platelet PGE 1 production is moderately reduced whereas in schizophrenia it is severely reduced.

4. Disorders of lipid and carbohydrate metabolism including diabetes mellitus and disorders in which blood chloesterol levels are elevated.

5. Disorders in which blood vessels go into spasm including agina pectoris, myocaridal infarction and Raynaud's syndrome; also hypertension.

6. Disorders of inflammation showing excessive production of 2-series PGs from arachidonic acid including disorders showing also low levels of cyclic AMP.

More particularly the conditions referred to in the inventor's prior patent applications referred to herein may be treated, such as schizophrenia, skin disorders, menstrual disorders and obesity as referred to in European Patent Specification No. A 0003407 (US Patent No. 4273763) and cancer and inflammatory disorders as referred to and listed in European Patent Specification No. A 000 4770 (US Patent No. 4309415) and sundry diseases including the effects of alcohol as referred to in European Patent Specification No. 0019423.

Selective enhancement of 1-series PG production has been explored in human platelets. The method is given in detail later herein but briefly human platelets are incubated with radioactive DGLA or

arachidonic acid. The PGs produced during incubation are extracted by conventional means and separated by thin layer chromatography, and the amount of radioactivity appearing in each PG or related substance is counted, PGE 1, PGF 1$\alpha$ and thromboxane B1 from DGLA and PGE 2, PGF 2$\alpha$ and thromboxane B2 from AA are estimated. The results, as given herein, demonstrate the inventor's belief that the effects of various agents on AA and DGLA conversion can be quite different and that it is possible to selectively enhance formation of PGE 1 and other 1-series PG compounds. The effect is believed to be by influencing the conversion of DGLA to the 1-series PGs.

The balance between 1-series and 2-series PGs is, the inventor believes, significant in terms of overall control of the conversion pathways given earlier. Such control is not understood in detail but without restriction to the theory it appears first that PGE 2 is able to enhance the formation of 1-series PGs and second that PGE 1 is able to block arachidonic acid mobilisation from tissue stores. Thus the condition for a negative feedback control loop exists; overproduction of PGE 2 from AA will activate PGE 1 synthesis, the PGE 1 will inhibit AA mobilisation, and production of 2-series PGs will drop. Further, TXA 2, an unstable product of the 2-series endoperoxides arising in 2-series PG production, also appears to enhance 1-series PG and in particular PGE 1 production. Thus again the activity of the 2-series PG synthesis pathway gives rise indirectly to a material that controls that pathway.

Effective agents

The inventor proposes the following agents for their beneficial effect on PG metabolism, namely natural or synthetic carotenoids or retinoids having, or giving rise in the body to compounds having, Vitamin A activity, and in particular $\beta$-carotene, other various isomers of retinoic acid, retinal and retinols, and their Vitamin A active derivatives.

The structure of the retinol form of Vitamin A is, for example;

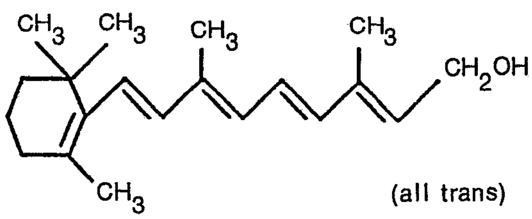

(all trans)

while $\beta$-carotene may be regarded as two such molecules joined by an ethylene group in place of the two —$CH_2OH$ groups.

Specific results on retinoic acid

Retinoic acid, chosen as a type compound representative of retinoids in general, has been tested for effect on PG metabolism in the human platelet system. The technique is given in detail below. Retinoic acid was incubated with platelets supplied with either 14C-AA or 14C-DGLA, control experiments being done without the retinoic acid. Results, with amounts expressed as percentages of the controls, were:

| | Precursor acid<br>Product | DGLA<br>PGE1 | DGLA<br>TXB1 | AA<br>PGE2 | AA<br>TXB2 |
|---|---|---|---|---|---|
| 1 $\mu$g/ml retinoic acid | | 110% | 114% | 98% | 96% |
| 10 $\mu$g/ml retinoic acid | | 118% | 121% | 81% | 77% |
| 100 $\mu$g/ml retinoic acid | | 157% | 153% | 63% | 62% |

Similar tests on retinol and retinal as other natural forms of Vitamin A have shown results of the same kind, enhancing DGLA conversion at concentrations which have no effect on AA conversion or actually reduce it.

Materials and methods

The detailed technique with platelets is given below by way of example.

(1-$^{14}$C) arachidonic acid and (1-$^{14}$C) dihomo-$\gamma$-linolenic acid were used, diluted with hexane to specific activities of about 5 $\mu$Ci/$\mu$mol. One day expired (2 days old) human platelets were obtained and used within 48 hours of expiration. One unit was centrifuged at 1000 g for 15 minutes and the supernatant drawn off. The platelet pellet was resuspended in Tris-NaCl-EDTA buffer, made up of 0.15 M NaCl, 0.15 M Tris HCl at pH 7.4 and 0.077 M NaEDTA (90:8:2 v/v/v). The platelets were recentrifuged, the supernatent removed and the pellet resuspended in Krebs-Henseleit buffer (without calcium) at pH 7.4. The washed platelet suspension contained about 1—2% red blood cells. All glassware used in the preparation of the platelets was siliconized.

Four equal sized 1 ml aliquots of the platelet suspension, containing $10^9$ platelets/ml were incubated with e.g. 0.5 $\mu$Ci $^{14}$C-DGLA for five minutes. At the beginning of the incubation the material

under test was added to the suspensions. The reaction was stopped after five minutes by addition of 1/10 volume of 10% formic acid. The suspension was then extracted three times with ethyl acetate and the fractions pooled and dried under vacuum. The extract was then taken up with 5 ml chloroform/methanol (2/1, v/v). Recovery of radioactive material in the extract was checked by taking 50 μl of the chloroform/methanol and counting by liquid scintillation. Recovery was in the range 80—95% in most experiments.

The chloroform/methanol extract was then reduced in volume to 1 ml under dry prepurified nitrogen. Thin layer chromatography was carried out on 500 μg precoated, prescored silica gel G Uniplates® (Analtech). Plates were activated by heating to 100°C for 1 hour immediately prior to use. The solvent system was chloroform:methanol:acetic acid:water (90:8:1:0.8). Reference compounds were run at the same time and visualised by phosphomolybdic acid spray followed by brief heating. The bands on the plates corresponding to the reference PGE 1, PGF 1α and TXB 1 were scraped off and eluted with 20 ml acetone. Each elution was then evaporated to dryness and counted by liquid scintillation (Beckman 100 LS counter).

Relationship to previous proposals

The above approaches may be used in combination with other materials as disclosed in the earlier patent applications of the inventor to which reference should be made, namely European Nos. 79300079.5, 79300546.3 and 80301510.6 (Publication Nos. 0 003 407, 0 004 770 and 0 019 423).

Among these materials are a number believed to act by enhancing mobilisation of DGLA reserves, including zinc, penicillin and β-lactam antibiotics generally, and also penicillamine, phenformin and levamisole when the other effects of these materials are acceptable.

Further, since there is evidence that thromboxane A2 may indirectly enhance formation of PGE 1, substances such as colchicine, amantadine and melatonin, and also griseofulvin, vinblastine, vincristine and interferon as discussed in the patent specifications referred to above and believed to act through increasing the production or effect of thromboxane A2, can also be used in conjunction with the materials of the present invention.

In European Specification No. 0 019 423 the further materials disclosed include Vitamin C, ethanol and opiate antagonists such as naloxone, again for this effect in PG metabolism.

As appears from the earlier patent specifications, in searching for ways to regulate PGE 1 formation the inventor has previously concentrated on the conversion of DGLA stores to free DGLA since this is believed to be a key rate-limiting step and since it has also been believed that factors which regulate conversion of free arachidonate to PGs will also regulate conversion of free DGLA to PGs. The present work has been more on the conversion of DGLA and of AA to the respective PGs, and as noted above it has been found that the factors regulating the two PG pathways are in some respects quite different. The discoveries on which the present application is based however build on and add to the earlier invention rather than superseding them.

Detailed statement of the present invention

In the light of the general discussion above, and of the fact that compositions containing, inter alia, γ-linolenic acid and Vitamin A have been on sale in "health food" outlets as dietary supplements for purchase by the general public, the present invention in its various aspects may be summarised as:

A. A composition for use in any condition in which enhancement of 1-series PG production, or more broadly influence of the 1-series/2-series PG balance in the body in favour of 1-series PGs, is indicated, and particularly any of the conditions listed earlier herein, comprising (a) γ-linolenic acid and/or dihomo-γ-linolenic acid, said acids being used if desired as physiologically functional derivatives thereof and (b) of the agents specified herein.

Dose ranges

Dose ranges in humans, initially individual doses, are for example as follows:

| | |
|---|---|
| Retinol | 50 μg to 300 mg/day preferably 3 to 15 mg/day |
| Retinoic acid | 1 mg to 3 g/day preferably 6 to 20 mg/day |
| β-Carotene | 1 mg to 6 g/day preferably 20 to 100 mg/day |

and biologically equivalent amounts of the Vitamin A active materials described herein.

Dose ranges for materials auxiliary to those of the invention are discussed elsewhere herein. All the materials may be given in doses of for example one half, one third or one quarter of the above amounts. The amounts are related to those quoted earlier for platelet and other experiments, though of course a precise relation cannot be given in view of variation in inactivation and excretion rates and volume of distribution.

Packs

If it is not desired to have compositions comprising the active materials together, as listed above, packs may be prepared comprising the materials presented for separate or part joint and part separate administration in the appropriate relative amounts, and use of such packs is within the purview of the invention.

Dietary compositions

The invention is chiefly described in terms of pharmaceutical compositions, but it will be understood that the γ-linolenic and other acids, being in the nature of dietary supplements, could be incorporated in a dietary margarine or other foodstuffs; such foodstuffs, possibly containing other active materials and generally referred to in this description as dietary or pharmaceutical compositions, are within the purview of the invention and thus of the term pharmaceutical compositions, packs or the like used in the claims.

Veterinary applications

It will be understood that where a disorder of the kind calling for treatment in animals arises, the invention while described primarily in terms of human medicine and treatment is equally applicable in the veterinary field.

Amounts of active materials (adjuncts to present invention)

Amounts of materials are:

| | |
|---|---|
| Zinc | 2.5 to 800 mg/day, preferably 10—80 mg calculated as zinc |
| β-lactam antibiotics | 0.5 to 10 g/day |
| Penicillamine | 50 mg to 10 g/day |
| Phenformin | 10 mg to 5 g/day |
| Levamisole | 10 mg to 2 g/day |
| Colchicine | 0.3 to 15 mg/day, preferably 0.6 to 2.4 mg |
| Melatonin | 10 mg to 5 g/day |
| Amantadine | 100 mg to 1000 mg/day |
| Griseofulvin | 0.5 to 5 g/day |
| Vinblastine | 0.5 to 5 mg/kg/week (average weight 70 kg) |
| Vincristine | 0.1 to 1.0 mg/kg/week (average weight 70 kg) |
| Interferon (by injection) | $1 \times 10^5$ to $1 \times 10^8$ units/day |
| Ascorbic acid | 50 mg to 50 g/day, preferably 250 mg to 5 g/day |
| Naloxone | 0.1 to 500 mg/day, for example orally or parenterally in 4 or 6 divided doses, preferably 10 mg to 200 mg/day |
| Nalorphine | 1 mg to 5 g/day as last, preferably 10 mg to 2 g/day |
| Levallorphan | 0.2 mg to 1 g/day as last |
| Ethanol | 5 to 500 ml/day, preferably 50 to 200 ml/day |

All the materials may be given in doses of for example one half, one third or one quarter of the above amounts. The amounts are related to those quoted earlier for platelet and other experiments, though of course a precise relation cannot be given in view of variation in inactivation and excretion rates and volume of distribution.

Detailed discussion of suitable amounts and forms of use is contained in the published patent specifications referred to earlier, to which reference may be made. In particular the β-lactam antibiotics are conveniently any of the known penicillin and cephalosporin antibiotics (including semi-synthetic antibiotics) such as, for example, penicillin G, penicillin N, penicillin V, cephalexin, cephalothin, ampicillin, amoxycillin, cloxacillin and cephaloglycin. Any of these may be used in the form of their

physiologically functional non-toxic derivatives, for example alkali metal salts, e.g. sodium and potassium salts, and salts with organic bases, and reference to an antibiotic herein includes reference to such derivatives.

Amount of $\gamma$-linolenic and other acids specifically

A preferred daily dosage for all purposes for an adult (weight ca 75 kg) is from 0.05 to 0.1 up to 1, 2, 5 or even 10 g as required of $\gamma$-linolenic acid or equivalent weight (calculated as $\gamma$-linolenic acid) of dihomo-$\gamma$-linolenic acid or physiologically functional derivative of either. Amounts in particular may be 0.1 to 1.0 g daily. Corresponding doses of the Oneothera oil containing 8 to 10% of $\gamma$-linolenic acid, are easily calculated. In place of, or in addition to, $\gamma$-linolenic acid, one may use dihomo-$\gamma$-linolenic acid or a physiologically functional derivative thereof, in amounts equivalent in molar terms to $\gamma$-linolenic acid and calculated as such. This dosage can for example be taken as a single dose or divided into 2, 3 or 4 subdivisions thereof as convenient.

Forms and sources of $\gamma$-linolenic and other acids

Convenient physiologically functional derivatives of $\gamma$-linolenic acid and dihomo-$\gamma$-linolenic acid for use according to the invention for all the purposes described include the $C_1$—$C_4$ alkyl (e.g. methyl) esters and the glycerides of the acids.

If desired, pharmaceutical compositions may be produced for use in the invention by associating natural or synthetic $\gamma$-linolenic acid (or a physiologically functional derivative thereof) and/or dihomo-$\gamma$-linolenic acid (or a physiologically functional derivative thereof), as such, with an acceptable pharmaceutical vehicle. It is at present convenient to incorporate the $\gamma$-linolenic acid into compositions in the form of an available oil having a high $\gamma$-linolenic acid content, hence references to "oil" herein.

At the present time known natural sources of oils having a high $\gamma$-linolenic acid content are few (there are no known natural sources of significant amounts of dihomo-$\gamma$-linolenic acid). One source of oils currently available is the seed of Evening Primrose species such as *Oenothera biennis L.* and *Oenothera lamarckiana*, the oil extract therefrom containing $\gamma$-linolenic acid (about 8%) and linoleic acid (about 72%) in the form of their glycerides together with other glycerides (percentages based on total fatty acids). Other sources of $\gamma$-linolenic acid are Borage species such as *Borago officinalis* which, though current yield per acre is low, provide a richer source of $\gamma$-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglycerides of $\gamma$-linolenic and linoleic as the main fatty acid components, the $\gamma$-linolenic acid content being if desired a major proportion. Seed oil extracts appear to have a stabilising effect upon any dihomo-$\gamma$-linolenic acid or physiologically functional derivative thereof.

Pharmaceutical presentation

The compositions according to the invention are conveniently in a form suitable for oral, rectal, parenteral or topical administration in a suitable pharmaceutical vehicle, as discussed in detail for example in Williams U.K. Patent Specification No. 1 082 624, to which reference may be made, and in any case very well known generally for any particular kind of preparation. Thus for example tablets, capsules, ingestible liquid or powder preparations, creams and lotions for topical application, or suppositories, can be prepared as required. Injectable solutions of hydrolysed Oenothera oil may be prepared using albumin to solubilise the free acid.

Advantageously a preservative is incorporated into the preparations, $\alpha$-Tocopherol in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions useful in treatment according to the invention:

Examples

Pharmaceutical compositions contain a unit dose of an oil extract from the seeds of *Oenothera biennis L.*, and of one of the active materials of the present invention, optionally with methyl dihomo-$\gamma$-linolenate and/or zinc oleate and/or penicillin V and/or any of the other active materials referred to herein directly or by cross reference to other patent applications of the inventor. They may be presented by encapsulation of the natural oil in soft gelatine capsules by known methods.

The oil is extracted from the seeds by one of the conventional methods of extraction such as cold pressure, screw pressure after partially cooking the seed, or solvent extraction.

Fractionation of a typical sample of this oil shows a yield of 97.0% oil in the form of methyl esters, with the relative proportions:

| | |
|---|---|
| Palmitate | 6.15 |
| Stearate | 1.6 |
| Oleate | 10.15 |
| Linoleate | 72.6 |
| γ-Linolenate | 8.9 |

As preservative, α-tocopherol is added to the oil in a concentration of 0.1%.

Gelatin capsules containing oil extracts prepared as described above, each having the following contents of active ingredients (0.5 g oil extract=ca 0.045 g γ-linolenic acid), are prepared in conventional fashion.

The following are specific examples of capsules that may be given in treatment of the conditions listed earlier.

Example 1

Capsules containing:

0.5 g Evening Primrose oil
3.0 mg Retinol

to be taken one capsule four times a day.

Example 2

Capsules containing:

0.5 g Evening Primrose oil
1.0 mg Retinol

one capsule to be taken eight times a day.

Similarly, capsules containing the oil with 5 to 20 mg β-carotene or 1 to 5 mg of retinoic acid can be taken; or for example 1 to 3 mg of retinol or 1 to 5 mg of retinoic acid, can be taken in capsules otherwise as described in the Examples of the earlier patent specifications mentioned herein and containing for example in addition to the oil and carotenoid or retinoid 10 mg zinc sulphate or 0.25 g penicillin V (European Specification No. 0003407); or 0.15 to 0.3 mg cholchicine, 100 mg amantadine, 25 mg lavamisole, 100 mg penicillamine, or the like (European Specification No. 0004770); or 200 mg ascorbic acid or 5 mg naloxone or the like (European Specification No. 0019423). Reference should be made to all other specifications for more details.

Further, topical preparations can be made, for example for use against acne, containing by weight from 0.01 to 0.1% of any of the active Vitamin A compounds, 0.1 to 25% Evening Primrose Oil, and base preparations to 100%.

**Claims for the Contracting States: BE CH DE FR GB IT LI NL SE LU**

1. A composition comprising (a) γ-linolenic acid and/or dihomo-γ-linolenic acid, said acid(s) being used if desired as physiologically acceptable salt, ester, or other derivative thereof convertible in the body thereto, and (b) one or more natural or synthetic carotenoids or retinoids having, or giving rise in the body to compounds having, Vitamin A activity, for use in influencing the 1-series/2-series PG balance in the body in favour of 1-series PGs.

2. A composition as in claim 1 wherein (b) comprises one or more materials selected from β-carotene, isomers of retinoic acid, retinal and retinol and Vitamin A active derivatives thereof.

3. A composition as in claim 2 presented for administration in quantities to give:

| | |
|---|---|
| Retinol | 50 μg to 300 mg/day preferably 3 to 15 mg/day |
| Retinoic acid | 1 mg to 3 g/day preferably 6 to 20 mg/day |
| β-Carotene | 1 mg to 6 g/day preferably 20 to 100 mg/day |

or equivalent amounts of other carotenoids or retinoids as referred to in claim 1.

4. A composition as in any preceding claim comprising further (i) one or more materials selected from zinc, β-lactam antibiotics, penicillamine, phenformin and levamisole, or (ii) one or more materials selected from colchicine, melatonin, amantadine, griseofulvin, vinblastine, vincristine and interferon, or (iii) one or more materials selected from ascorbic acid, naloxone, nalorphine, levallorphan and ethanol.

5. A composition as in claim 4 represented for administration in quantities to give:

| | |
|---|---|
| Zinc | 2.5 to 800 mg/day |
| β-lactam antibiotics | 0.5 to 10 g/day |
| Penicillamine | 50 mg to 10 g/day |
| Phenformin | 10 mg to 5 g/day |
| Levamisole | 10 mg to 2 g/day |
| Colchicine | 0.3 to 15 mg/day |
| Melatonin | 10 mg to 5 g/day |
| Amantadine | 100 mg to 1000 mg/day |
| Griseofulvin | 0.5 to 5 g/day |
| Vinblastine | 0.5 to 5 mg/kg/week |
| Vincristine | 0.1 to 1.0 mg/kg/week |
| Interferon (by injection) | $1 \times 10^5$ to $1 \times 10^8$ units/day |
| Ascorbic acid | 50 mg to 50 g/day |
| Naloxone | 0.1 to 500 mg/day |
| Nalorphine | 1 mg to 5 g/day |
| Levallorphan | 0.2 mg to 1 g/day |
| Alcohol | 5 to 500 ml/day |

6. A composition as in any preceding claim presented for administration to give 0.5 to 10 g daily of γ-linolenic acid or equivalent amount, calculated as γ-linolenic acid, of dihomo-γ-linolenic acid or physiologically acceptable salt, ester or other derivative of either convertible in the body thereto.

7. A composition as in any preceding claim, for use in therapy specifically of:

1. Defective T lymphocyte function including such defective function as allergic and inflammatory disorders, multiple sclerosis, schizophrenia and cancer;
2. Defective regulation of collagent formation and breakdown including such defective regulation as rheumatoid arthritis, systemic lupus erythematosus, Crohn's disease and the "collagen" diseases;
3. Mental illnesses showing low PGE 1 levels including depression, schizophrenia and alcohol withdrawal and the later stages of alcohol intoxication;
4. Disorders of lipid and carbohydrate metabolism including diabetes mellitus and disorders in which blood cholesterol levels are elevated;
5. Disorders in which blood vessels go into spasm including angina pectoris, myocardial infarction and Raynaud's syndrome; also hypertension;
6. Disorders of inflammation showing excessive production of 2-series PGs from arachidonic acid including disorders showing also low levels of cyclic AMP.

8. A pack comprising the components of the composition of any one of claims 1 to 6 presented for conjoint administration, for the uses set out in claim 1 or claim 7.

**Claims for the Contracting State: AT**

1. The use of (a) γ-linolenic acid and/or dihomo-γ-linolenic acid, said acid(s) being used if desired as physiologically acceptable salt, ester or other derivative thereof convertible in the body thereto, and (b) one or more natural or synthetic carotenoids or retinoids having, or giving rise in the body to compounds having, Vitamin A activity, for the method of producing a composition for influencing the 1-series/2-series PG balance in the body in favour of 1-series PGs.

2. The use according to claim 1 wherein the composition is a composition as in claim 1 wherein (b) comprises one or more materials selected from β-carotene, isomers of retinoic acid, retinal and retinol and Vitamin A active derivatives thereof.

3. The use according to claim 1 wherein the composition is a composition as in claim 2 presented for administration in quantities to give:

| | |
|---|---|
| Retinol | 50 $\mu$g to 300 mg/day preferably 3 to 15 mg/day |
| Retinoic acid | 1 mg to 3 g/day preferably 6 to 20 mg/day |
| $\beta$-Carotene | 1 mg to 6 g/day preferably 20 to 100 mg/day |

or equivalent amounts of other carotenoids or retinoids as referred to in claim 1.

4. The use according to claim 1 wherein the composition is a composition as in any preceding claim comprising further (i) one or more materials selected from zinc, $\beta$-lactam antibiotics, penicillamine, phenformin and levamisole, or (ii) one or more materials selected from colchicine, melatonin, amantadine, griseofulvin, vinblastine, vincristine and interferon, or (iii) one or more materials selected from ascorbic acid, naloxone, nalorphine, levallorphan and ethanol.

5. The use according to claim 1 wherein the composition is a composition as in claim 4 presented for administration in quantities to give:

| | |
|---|---|
| Zinc | 2.5 to 800 mg/day |
| $\beta$-lactam antibiotics | 0.5 to 10 g/day |
| Penicillamine | 50 mg to 10 g/day |
| Phenformin | 10 mg to 5 g/day |
| Levamisole | 10 mg to 2 g/day |
| Colchicine | 0.3 to 15 mg/day |
| Melatonin | 10 mg to 5 g/day |
| Amantadine | 100 mg to 1000 mg/day |
| Griseofulvin | 0.5 to 5 g/day |
| Vinblastine | 0.5 to 5 mg/kg/week |
| Vincristine | 0.1 to 1.0 mg/kg/week |
| Interferon (by injection) | $1 \times 10^5$ to $1 \times 10^8$ units/day |
| Ascorbic acid | 50 mg to 50 g/day |
| Naloxone | 0.1 to 500 mg/day |
| Nalorphine | 1 mg to 5 g/day |
| Levallorphan | 0.2 mg to 1 g/day |
| Alcohol | 5 to 500 ml/day |

6. The use according to claim 1 wherein the composition is a composition as in any preceding claim presented for administration to give 0.5 to 10 g daily or $\gamma$-linolenic acid or equivalent amount, calculated as $\gamma$-linolenic acid, or dihomo-$\gamma$-linolenic acid or physiologically acceptable salt, ester or other derivative of either, convertible in the body thereto.

**Patentansprüche für die Vertragsstaaten: BE CH DE FR GB IT LI LU NL SE**

1. Präparat, bestehend aus (a) $\gamma$-Linolensäure und/oder Dihomo-$\gamma$-linolensäure, die nach Bedarf in Form von physiologisch aufnehmbarem Salz, Ester oder sonstigem, im Körper hierzu umsetzbarem Derivat der Säure(n) verwendet wird (werden), und (b) mindestens einem natürlichen oder synthetischen Carotinoid oder Retinoid, welche Vitamin-A-Aktivität besitzen oder im Körper zum Entstehen von Verbindungen mit Vitamin-A-Aktivität führen, zur Verwendung im Sinne einer

Beeinflussung des Serie 1/Serie 2-Prostaglandin-Gleichgewichts im Körper zugunsten der Serie 1-Prostaglandine.

2. Präparat nach Anspruch 1, dadurch gekennzeichnet, daß (b) mindestens einen der Stoffe β-Carotin, Isomere von Retinsäure, Retinal und Retinol und deren Derivate mit Vitamin-A-Aktivität enthält.

3. Präparat nach Anspruch 2, dargeboten zur Verabreichung in Mengen, die

| | |
|---|---|
| Retinol | 50 μg bis 300 mg/Tag, vorzugsweise 3 bis 15 mg/Tag |
| Retinsäure | 1 mg bis 3 g/Tag, vorzugsweise 6 bis 20 mg/Tag |
| β-Carotin | 1 mg bis 6 g/Tag, vorzugsweise 20 bis 100 mg/Tag |

oder gleichwertige Mengen anderer Carotinoide oder Retinoide nach den Angaben in Anspruch 1 ergeben.

4. Präparat nach einem der vorhergehenden Ansprüche, außerdem bestehend aus: (I) mindestens einem der Stoffe Zink, β-Lactam-Antibiotika, Penicillamin, Phenformin und Levamisol oder (II) mindestens einem der Stoffe Colchicin, Melatonin, Amantadin, Griseofulvin, Vinblastin, Vincristin und Interferon oder (III) mindestens einem der Stoffe Ascorbinsäure, Naloxon, Nalorphin, Levallorphan und Ethanol.

5. Präparat nach Anspruch 4, dargeboten zur Verabreichung in solchen Mengen, daß sich ergeben:

| | |
|---|---|
| Zink | 2,5 bis 800 mg/Tag |
| β-Lactam-Antibiotika | 0,5 bis 10 g/Tag |
| Penicillamin | 50 mg bis 10 g/Tag |
| Phenformin | 10 mg bis 5 g/Tag |
| Levamisol | 10 mg bis 2 g/Tag |
| Colchicin | 0,3 bis 15 mg/Tag |
| Melatonin | 10 mg bis 5 g/Tag |
| Amantadin | 100 mg bis 1000 mg/Tag |
| Griseofulvin | 0,5 bis 5 g/Tag |
| Vinblastin | 0,5 bis 5 mg/kg/Woche |
| Vincristin | 0,1 bis 1,0 mg/kg/Woche |
| Interfero (Injekt.) | $1 \cdot 10^5$ bis $1 \cdot 10^8$ Einh./Tag |
| Ascorbinsäure | 50 mg bis 50 g/Tag |
| Naloxon | 0,1 bis 500 mg/Tag |
| Nalorphin | 1 mg bis 5 g/Tag |
| Levallorphan | 0,2 mg bis 1 g/Tag |
| Alkohol | 5 bis 500 ml/Tag |

6. Präparat nach einem der vorhergehenden Ansprüche, dargeboten zur Verabreichung, so daß sich 0,5 bis 10 g/Tag γ-Linolensäure oder eine gleichwertige Menge, errechnet auf der Basis von γ-Linolensäure, von Dihomo-γ-linolensäure oder physiologisch aufnehmbaren Salz, Ester oder sonstigem im Körper hierzu umsetzbarem Derivat einer der beiden ergeben.

7. Präparat nach einem der vorhergehenden Ansprüche zur therapeutischen Verwendung, insbesondere bei:

1. Mangelhafter Lyphozytenfunktion, einschließlich mangelhafter Funktion wie allergische und entzündliche Störungen, Multiple Sklerose, Schizophrenie, und Krebs;

2. Mangelhafte Regulation der Kollagenbildung und des Kollagenabbaus, einschließlich solcher mangelhafter Regulation wie rheumatoide Arthritis, systemischer Lupus erythematosus, Enteritis regionalis und die "Kollagen"-Krankheiten;

3. Geisteskrankheiten mit niedrigem PGE-1-Niveau, einschließlich Depression, Schizophrenie und Alkohol-Entziehung und die späteren Stadien der Alkoholvergiftung;

4. Störungen des Lipoid-und Kohlenhydrat-Stoffwechsels, einschließlich Diabetes Mellitus, und Störungen, bei denen der Cholesterinspiegel erhöht ist;

5. Störungen, bei denen Blutgefäße Krampfzustände erleiden, einschließlich Angina pectoris, Myocardinfarkt und Raynaud-Syndrom, ebenso Hypertonie;

6. Entzündungsstörungen mit übermäßiger Erzeugung von Serie 2-Prostaglandinen von Arachidonsäure, einschließlich Störungen, die auch niedrige cAMP-Spiegel zeigen.

8. Packung, enthaltend die Bestandteile des Präparats nach einem der Ansprüche 1 bis 6, dargeboten zur gemeinsamen Verabreichung, zu den in den in den Ansprüchen 1 und 7 genannten Zwecken.

**Patentansprüche für der Vertragsstaat: AT**

1. Verwendung von (a) $\gamma$-Linolensäure und/oder Dihomo-$\gamma$-linolensäure, die nach Bedarf in Form von physiologisch aufnehmbarem Salz, Ester oder sonstigem, im Körper hierzu umsetzbarem Derivat der Säure(n) verwendet wird (werden), und (b) mindestens einem natürlichen oder synthetischen Carotinoid oder Retinoid, welche Vitamin-A-Aktivität besitzen oder im Körper zur Entstehung von Verbindungen mit Vitamin-A-Aktivität führen, für das Verfahren zum Herstellen einer Verbindung zur Beeinflussung des Serie 1/Serie 2-Prostaglandin-Gleichgewichts zugunsten der Serie 1-Prostaglandine.

2. Verwendung nach Anspruch 1, dadurch gekennzeichnet, daß das Präparat ein Präparat nach Anspruch 1 darstellt, wobei (b) mindstens einen der Stoffe $\beta$-Carotin, Isomere der Retinsäure, Retinal und Retinol und deren Derivate mit Vitamin-A-Aktivität enthält.

3. Verwendung nach Anspruch 1, dadurch gekennzeichnet, daß das Präparat ein Präparat nach Anspruch 2 darstellt, dargeboten zur Verabreichung in Mengen, die

| | |
|---|---|
| Retinol | 50 $\mu$g bis 300 mg/Tag, vorzugsweise 3 bis 15 mg/Tag |
| Retinsäure | 1 mg bis 3 g/Tag, vorzugsweise 6 bis 20 mg/Tag |
| $\beta$-Carotin | 1 mg bis 6 g/Tag, vorzugsweise 20 bis 100 mg/Tag |

oder gleichwertige Mengen anderer Carotinoide oder Retinoide nach den Angaben im Anspruch 1 ergeben.

4. Verwendung nach Anspruch 1, dadurch gekennzeichnet, daß das Präparat ein Präparat nach einem der vorhergehenden Ansprüche darstellt, und daß es ferner enthält: (I) mindestens einen der Stoffe Zink, $\beta$-Lactam-Antibiotika, Penicillamin, Phenformin und Levamisol, oder (II) mindestens einen der Stoffe Colchicin, Melatonin, Amantadin, Griseofulvin, Vinblastin, Vincristin und Interferon, oder (III) einen der Stoffe Ascorbinsäure, Naloxon, Nalorphin, Levallorphan und Ethanol.

5. Verwendung nach Anspruch 1, dadurch gekennzeichnet, daß das Präparat ein Präparat nach Anspruch 4 darstellt, das zur Verabreichung in solchen Mengen dargeboten wird, daß sich ergeben:

| | |
|---|---|
| Zink | 2,5 bis 800 mg/Tag |
| $\beta$-Lactam-Antibiotika | 0,5 bis 10 g/Tag |
| Penicillamin | 50 mg bis 10 g/Tag |
| Phenformin | 10 mg bis 5 g/Tag |
| Levamisol | 10 mg bis 2 g/Tag |
| Colchicin | 0,3 bis 15 mg/Tag |
| Melatonin | 10 mg bis 5 g/Tag |
| Amantadin | 100 mg bis 1000 mg/Tag |
| Griseofulvin | 0,5 bis 5 g/Tag |
| Vinblastin | 0,5 bis 5 mg/kg/Woche |

| | |
|---|---|
| Vincristin | 0,1 bis 1,0 mg/kg/Woche |
| Interferon (Injekt.) | $1 \cdot 10^5$ bis $1 \cdot 10^8$ Einh./Tag |
| Ascorbinsäure | 50 mg bis 50 g/Tag |
| Naloxon | 0,1 bis 500 mg/Tag |
| Nalorphin | 1 mg bis 5 g/Tag |
| Levallorphan | 0,2 mg bis 1 g/Tag |
| Alkohol | 5 bis 500 ml/Tag |

6. Verwendung nach Anspruch 1, dadurch gekennzeichnet, daß das Präparat ein Präparat nach einem der vorhergehenden Ansprüche darstellt, das zur Verabreichung in der Weise dargeboten wird, daß sich 0,5 bis 10 g/Tag $\gamma$-Linolensäure oder eine gleichwertige Menge, errechnet auf der Basis von $\gamma$-Linolensäure, von Dihomo-$\gamma$-linolensäure oder physiologisch aufnehmbarem Salz, Ester oder sonstigem, im Körper hierzu umsetzbaren Derivat einer der beiden ergeben.

**Revendications pour les Etats Contractants: BE CH DE FR GB IT LI LU NL SE**

1. Compositions caractérisées en ce qu'elles contiennent (a) de l'acide $\gamma$-linolénique et/ou de l'acide dihomo-$\gamma$-linolénique, lesdits acides étant utilisés, si on le désire, sous la forme d'un sel, ester, ou autre dérivé physiologiquement acceptable et convertible dans l'organisme en ledit acide, et (b) un ou plusieurs composés choisis parmi les rétinoïdes ou caroténoïdes naturels ou synthétiques ayant, ou donnant lieu dans l'organisme, à des composés ayant une activité de vitamine A, ces compositions étant utiles pour influencer l'équilibre des PG de série 1 par rapport aux PG de série 2 dans l'organisme, en faveur des PG de série 1.

2. Compositions selon la revendication 1, caractérisées en ce que le composant (b) comprend un ou plusieurs produits choisis parmi le $\beta$-carotène, les isomères de l'acide rétinoïque, le rétinal et le rétinol, ainsi que les dérivés de ces composés à activité de vitamine A.

3. Compositions selon la revendication 2, présentées pour l'administration en quantités telles qu'elles donnent:

| | |
|---|---|
| Rétinol | 50 $\mu$g à 300 mg/j, de préférence 3 à 15 mg/j |
| Acide rétinoïque | 1 mg à 3 g/j, de préférence 6 à 20 mg/j |
| $\beta$-carotène | 1 mg à 6 g/j, de préférence 20 à 100 mg/j |

ou des quantités équivalentes d'autres caroténoïdes ou rétinoïdes comme revendiqué dans la revendication 1.

4. Compositions selon l'une quelconque des revendications 1 à 3, caractérisées en ce qu'elles comprennent de plus (i) un ou plusieurs produits choisis parmi le zinc, les antibiotiques du type $\beta$-lactame, la pénicillamine, la phenformine et le lévamisole, ou (ii) un ou plusieurs produits choisis parmi: colchicine, mélatonine, amantadine, griséofulvine, vinblastine, vincristine et interféron, ou (iii) un ou plusieurs matériaux choisis parmi: acide ascorbique, naloxone, nalorphine, lévallorphane et éthanol.

5. Compositions selon la revendication 4, présentées pour l'administration en quantités telles qu'elles donnent:

| | |
|---|---|
| Zinc | 2,5 à 800 mg/j |
| Antibiotiques du type $\beta$-lactame | 0,5 à 10 g/j |
| Pénicillamine | 50 mg à 10 g/j |
| Phenformine | 10 mg à 5 g/j |
| Lévamisole | 10 mg à 2 g/j |
| Colchicine | 0,3 à 5 mg/j |
| Mélatonine | 10 mg à 5 g/j |
| Amantadine | 100 mg à 1000 mg/j |

| | |
|---|---|
| Griséolfulvine | 0,5 à 5 g/j |
| Vinblastine | 0,5 à 5 mg/kg/semaine |
| Vincristine | 0,1 à 1,0 mg/kg/semaine |
| Interféron (par injection) | $1\times10^5$ à $1\times10^8$ unités/j |
| Acide ascorbique | 50 mg à 50 g/j |
| Naloxone | 0,1 à 500 mg/j |
| Nalorphine | 1 mg à 5 g/j |
| Lévallorphane | 0,2 mg à 1 g/j |
| Alcool | 5 à 500 ml/j |

6. Compositions selon l'une quelconque des revendications 1 à 5, présentées pour l'administration de manière à donner 0,5 à 10 g/j d'acide $\gamma$-linolénique ou une quantité équivalente, calculée en acide $\gamma$-linolénique, d'acide dihomo-$\gamma$-linolénique ou d'un sel, ester ou autre dérivé physiologiquement acceptable d'un de ses composés, convertible dans l'organisme en l'un de ces acides.

7. Compositions selon l'une quelconque des revendications 1 à 6, utiles spécifiquement pour le traitement de:

1. Défaut de fonction de lymphocytes T, y compris des fonctions déficientes comme en cas de désordres allergiques et inflammatoires, de sclérose multiple, de schizophrénie et de cancer.
2. Régulation déficiente de la formation de collagène et de dégradation du collagène, y compris une régulation déficiente comme dans le cas de l'arthrite rhumatoïde, du lupus erythematosus systémique, de la maladie de Crohn, et des "maladies du collagène".
3. Maladies mentales impliquant des taux faibles de PGE 1, y compris la dépression, la schizophrénie et la suppression d'alcool, ainsi que les stades avancés de l'intoxiation alcoolique. Dans les cas de dépression, par exemple, la production de PGE 1 des plaquettes est modérément réduite, tandis que, dans le cas de schizophrénie, cette production est fortement réduite.
4. Désordres du métabolisme des lipides et des hydrates de carbone, y compris le mellitus des diabètes, et les désordres dans lesquels on note une augmentation des taux de cholestérol.
5. Désordres dans lesquels les vaisseaux sanguins subissent un spasme, y compris angina pectoris, l'infarctus du myocarde et le syndrome de Raynaud; également hypertension.
6. Désordres inflammatoires impliquant une production excessive de PG de la série 2 à partir d'acide arachidonique, et y compris les désordres impliquant également des taux trop faibles de AMP cyclique.

8. Coffrets, caractérisés en ce qu'ils comprennent les composants des compositions selon l'une quelconque des revendications 1 à 6, présentés pour administration conjointe, pour les applications décrites dans la revendication 1 ou la revendication 7.

**Revendications pour l'Etat Contractant: AT**

1. L'utilisation de (a) l'acide $\gamma$-linolénique et/ou de l'acide dihomo-$\gamma$-linolénique, lesdits acides étant utilisés, si on le désire, sous la forme d'un sel, ester, ou autre dérivé physiologiquement acceptable et convertible dans l'organisme en ledit acide, et (b) un ou plusieurs composés choisis parmi les rétinoïdes ou caroténoïdes naturels ou synthétiques ayant, ou donnant lieu dans l'organisme, à des composés ayant une activité de vitamine A, ces compositions étant utiles pour influencer l'équilibre des PG de série 1 par rapport aux PG de série 2 dans l'organisme, en faveur des PG de série 1.

2. L'utilisation selon la revendication 1, caractérisée en ce que les compositions sont celles de la revendication 1, dans lesquelles (b) comprend un ou plusieurs produits choisis parmi le $\beta$-carotène, les isomères de l'acide rétinoïque, le rétinal et le rétinol, ainsi que les dérivés de ces composés à activité de vitamine A.

3. L'utilisation selon la revendication 1, caractérisée en ce que les compositions sont celles de la revendication 2, présentées pour l'administration en quantités telles qu'elle donnent:

| | |
|---|---|
| Rétinol | 50 $\mu$g à 300 mg/j, de préférence 3 à 15 mg/j |
| Acide rétinoïque | 1 mg à 3 g/j, de préférence 6 à 20 mg/j |
| $\beta$-carotène | 1 mg à 6 g/j, de préférence 20 à 100 mg/j |

ou des quantités équivalentes d'autres caroténoïdes ou rétinoïdes comme revendiqué dans la revendication 1.

4. L'utilisation selon la revendication 1, caractérisée en ce que les compositions sont des compositions selon l'une quelconque des revendications précédentes et comprenant de plus (i) un ou plusieurs produits choisis parmi le zinc, les antibiotiques du type $\beta$-lactame, la pénicillamine, la phenformine et le lévamisole, ou (ii) un ou plusieurs produits choisis parmi: colchicine, mélatonine, amantadine, griséofulvine, vinblastine, vincristine et interféron, ou (iii) un ou plusieurs matériaux choisis parmi: acide ascorbique, naloxone, nalorphine, lévallorphane et éthanol.

5. L'utilisation selon la revendication 1, caractérisée en ce que les compositions sont celles de la revendication 4, présentées pour l'administration en quantités telles qu'elles donnent:

| | |
|---|---|
| Zinc | 2,5 à 800 mg/j |
| Antibiotiques du type $\beta$-lactame | 0,5 à 10 g/j |
| Pénicillamine | 50 mg à 10 g/j |
| Phenformine | 10 mg à 5 g/j |
| Lévamisole | 10 mg à 2 g/j |
| Colchicine | 0,3 à 15 mg/j |
| Mélatonine | 10 mg à 5 g/j |
| Amantadine | 100 mg à 1000 mg/j |
| Griséofulvine | 0,5 à 5 g/j |
| Vinblastine | 0,5 à 5 mg/kg/semaine |
| Vincristine | 0,1 à 1,0 mg/kg/semaine |
| Interféron (par injection) | $1\times10^5$ à $1\times10^8$ unités/j |
| Acide ascorbique | 50 mg à 50 g/j |
| Naloxone | 0,1 à 500 mg/j |
| Nalorphine | 1 mg à 5 g/j |
| Lévallorphane | 0,2 mg à 1 g/j |
| Alcool | 5 à 500 ml/j |

6. L'utilisation selon la revendication 1, caractérisée en ce que les compositions sont celles de l'une quelconque des revendications précédentes, présentées pour l'administration de manière à donner 0,5 à 10 g/j d'acide $\gamma$-linolénique ou une quantité équivalente, calculée en acide $\gamma$-linolénique, d'acide dihomo-$\gamma$-linolénique ou d'un sel, ester ou autre dérivé physiologiquement acceptable d'un de ses composés, convertible dans l'organisme en l'un de ces acides.

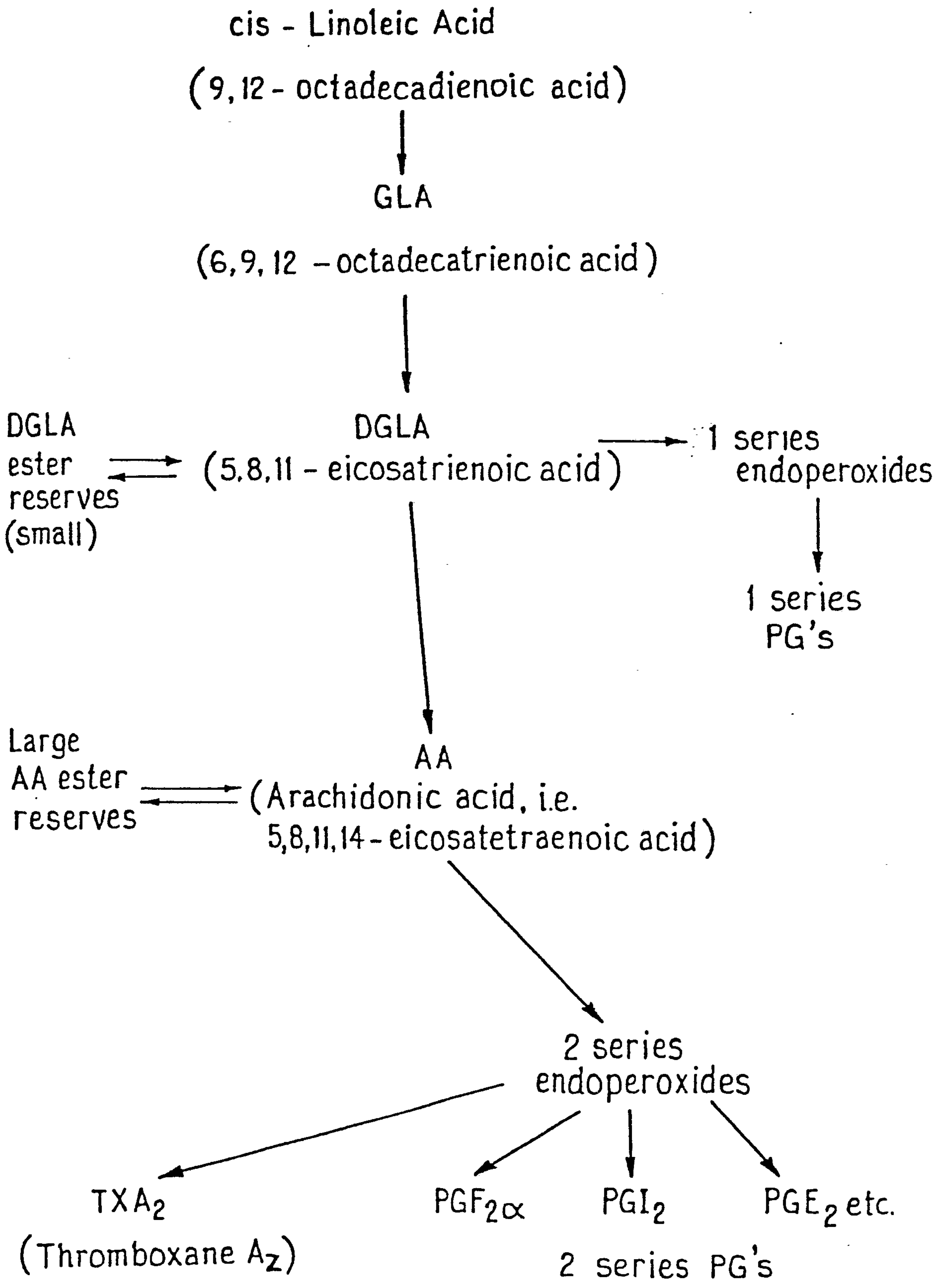

cis - Linoleic Acid
(9,12 - octadecadienoic acid)
GLA
(6,9,12 - octadecatrienoic acid)
DGLA ester reserves (small)
DGLA
(5,8,11 - eicosatrienoic acid)
1 series endoperoxides
1 series PG's
Large AA ester reserves
AA
(Arachidonic acid, i.e. 5,8,11,14 - eicosatetraenoic acid)
2 series endoperoxides
TXA2
(Thromboxane A2)
PGF2α
PGI2
PGE2 etc.
2 series PG's